# EUROPEAN PATENT APPLICATION

(11) **EP 0 689 851 A1**
(43) Date of publication of application: **03.01.1996**
(21) Application number: 95201782.0
(22) Date of filing: 29.06.1995
(51) Int. Cl.: A61M 25/01

(54) **Controlled flexible catheter**

(30) Priority: 01.07.1994 NL 9401107
(71) Applicant: CORDIS EUROPA N.V., NL-9301 LJ Roden (NL)
(72) Inventor: Van Muiden, Johannes Gerardus M., NL-9321 AZ Peize (NL); Mous, Frans, NL-9302 HP Drachten (NL)
(74) Representative: 't Jong, Bastiaan Jacobus

(57) **Abstract**

The invention relates to a catheter comprising a tube-like basic body with a proximal and a distal end and at least one lumen, a pull wire connected with the basic body close to the distal end and extending through the lumen to the proximal end. The basic body has, for at least part of a section close to the distal end, a varying stiffness in the circumferential direction, and wherein the section close to the distal end also has a varying stiffness in the longitudinal direction.

## Description

The invention relates to a catheter comprising a tube-like basic body with a proximal and a distal end, and at least one lumen. A pull wire has been received in the lumen of the catheter which engages close to the distal end. By pulling the pull wire the catheter will bend, as a result of which it is easier to place the catheter in a specific position inside the body of the patient.

The object of the invention is to improve such a catheter, e.g. known from WO 93/01857.

This aim is achieved with the catheter as characterised in claim 1. Because of the varying stiffness in the circumferential direction and in longitudinal direction, the bending performance of the catheter is so to speak "preprogrammed". By pulling the pull wire the catheter will always bend in the shape for which the minimum amount of energy is required.

Because of the invention it is possible to make a catheter bend into any desired, both two- and three-dimensional shape. When "programming" the curve, one only needs to ensure that in each section of the catheter in which a certain curve is required, the bending stiffness of the material of the catheter which in the bent state will be situated on the outside of the curve, will be greater than that on the inside of the curve.

Due to the varying stiffness in longitudinal direction, it is possible to preprogram, in addition to the desired bending direction, also the relative curve of certain parts of the catheter. A section with a more limited bending stiffness than another section in a longitudinal direction, will be curved more than the section with a greater bending stiffness, when a tensile force is applied to the pull wire. The direction of the curve obviously depends on the, in a circumferential direction varying stiffness of the sections concerned. Thus the more pliable section can bend in one direction and the stiffer section in an opposite direction, so that an S-bend is formed. Any desired combination of curves is achievable according to the invention.

A suitable embodiment is characterised in claim 2. The strip of material with the differing bending stiffness can for instance be incorporated in the basic body by coextrusion of materials with different bending stiffness properties.

With known flexible catheters the pull wire has been attached eccentrically close to the end in order to obtain the desired curve. With the invention it is however possible in a suitable manner to employ the measure as set out in claim 4. Even with very thin catheters the desired bending performance can be achieved.

A suitable embodiment is characterised in claim 5. The pull wire can be fixed securely to the end cap and the end cap can, during a later stage of the manufacturing process of the catheter, be fixed to the distal end of the basic body.

The invention will be explained in greater detail in the following description with reference to the attached drawings.
- Fig. 1: shows a partly cut away view of the end-section of a catheter according to the invention.
- Fig. 2: shows the cross-section at II-II in fig. 1.
- Fig. 3: shows an alternative of fig. 2.

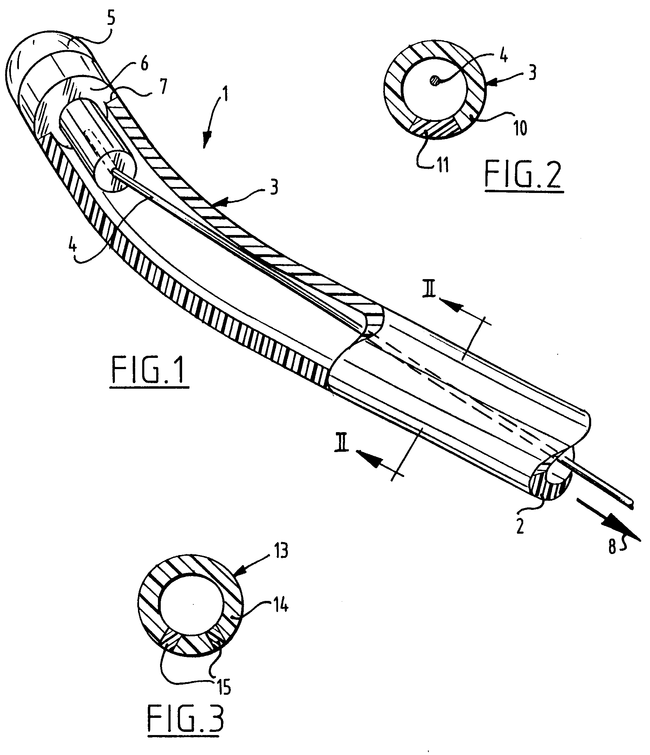

The catheter 1 of fig. 1 comprises a tube-like basic body 2 with one lumen. In fig. 1 the section of the catheter 1 close to the distal end has been illustrated. The proximal end is, in the usual manner, provided with connecting members not shown here, and for instance a control means for the pull wire 4 received in the catheter 1. This pull wire 4 has been attached with its distal end inside a stepped cap 5, which comprises a shoulder 6 which is placed against the end surface 7 of the basic body 2. The cap is provided with a rounded tip so that, on introduction of the catheter, no trauma will be caused.

The end-section 3 of the basic body 2 has according to the invention been made in such a way, that it has a varying stiffness in a circumferential direction.

As is shown in fig. 2, this has been realised in this embodiment by incorporating a strip of material 11 extending in the longitudinal direction in the wall of the basic body 2 with a bending stiffness different to that of the other material 10. During the manufacture of the tubular body 2, this strip 11 can for instance be formed inside it by means of coextrusion with the material 10.

When the material 11 has a greater bending stiffness than the material 10, the end-section 3 will preferably bend in such a direction that the material 11 will be deformed minimally, ie will be positioned on the outside of the curve when bent. When applying a tensile force in the direction of arrow 8 on the pull wire 4, the end-section 3 will always bend in the direction shown in fig. 1.

The varying stiffness in a circumferential direction can be achieved in different ways.

Fig. 3 illustrates an alternative embodiment wherein two strip-shaped sections 15, with a different bending stiffness compared to that of the material 14, have been received in the end-section 13. With this embodiment a more stable curve of the catheter in one plane is achieved.

It will be clear that when the strip 11 or the strips 15 are situated for part of a first section of the end-section on one side of the basic body and for part of another, possible adjoining second section, on the opposite side, an S-bend will be formed when applying a tensile force to the pull wire 4. In addition to such two-dimensional curves it is obviously also possible to allow for a three-dimensional bending performance by means of a suitable distribution of the stiffer and the more flexible material respectively in the circumferential- and longitudinal direction of the catheter.

When the bending stiffness varying in the circumferential direction is obtained by coextrusion of different materials, a rotating extrusion head on the extrusion machine can be employed in a suitable manner. By rotating the extrusion head, the desired distribution in the circumferential- and longitudinal direction of the catheter will be achieved. At the same time a gradual transition between the different sections in the longitudinal direction of the catheter is achieved in the form of a helical line, preventing kinks at certain places along the catheter.

## Claims

1. Catheter comprising a tube-like basic body with a proximal and a distal end and at least one lumen, a pull wire connected with the basic body close to the distal end and extending through the lumen to the proximal end wherein the basic body has, for at least part of a section close to the distal end, a varying stiffness in the circumferential direction and wherein the section close to the distal end also has a varying stiffness in the longitudinal direction.

2. Catheter as claimed in claim 1, wherein the section close to the distal end has at least one strip of material with a bending stiffness different to that of the other material, received in the wall of the basic body, extending in the longitudinal direction of the basic body.

3. Catheter as claimed in claim 2, wherein the strip has a greater bending stiffness.

4. Catheter as claimed in one of the previous claims, wherein the pull wire has been fixed in a central position in the cross-section of the basic body.

5. Catheter as claimed in claim 4, wherein the pull wire has been attached to an end cap which is positioned with a supporting surface against the end surface of the basic body.
